# FASCICULE DE BREVET EUROPEEN

(11) **EP 0 334 005 B1**
(45) Date de publication et mention de la délivrance du brevet: **12.04.1995**
(21) Numéro de dépôt: 89102016.6
(22) Date de dépôt: 06.02.1989
(51) Int. Cl.: C11B 9/00, A61K 7/46, C07C 43/00

(54) **Utilisation de 2-méthoxy-4-propyl-1-cyclohexanol comme agent parfumant**
Verwendung von 2-Methoxy-4-Propyl-1-Cyclohexanol als Duftstoff
Use of 2-methoxy-4-propyl-1-cyclohexanol as a perfuming agent

(30) Priorité: 25.02.1988 CH 701/88
(43) Date de publication de la demande: 27.09.1989
(73) Titulaire: FIRMENICH SA, CH-1211 Genève 8 (CH)
(72) Inventeur: Maillefer, Eric, CH-1026 Echandens (CH)
(74) Mandataire: Salvadori, Giuseppe

(56) Documents cités:
- CHEMICAL ABSTRACTS, vol. 83, 1975, page 224, column 2, abrégé no. 62164d,Columbus, Ohio, US; S.W. EACHUS et al.: "Hydrogenation of lignin model compounds in the presence of a homogeneous catalyst", & HOLZFORSCHUNG 1975,29(2), 41-8
- CHEMICAL ABSTRACTS, vol. 81, 1974, page 103, abrégé no. 79577x, Columbus, Ohio,US; W. SCHWEERS et al.: "Hydrogenolysis of lignin. V. Comparative hydrogenation of 4-alkylsyringoles", & HOLZFORSCHUNG 1974, 28(1), 20-4
- CHEMICAL ABSTRACTS, vol. 71, 1969, pages 324,325, abrégé no. 90976r, Columbus,Ohio, US; W. SCHWEERS: "Hydrogenolysis of lignin. III. Comparative hydration of 4-alkylphenols and 4-alkylguiacols", & HOLZFORSCHUNG 1969, 23(4), 120-7

## Description

La présente invention a trait au domaine de la parfumerie. Elle concerne plus particulièrement un dérivé du cyclohexanol de formule 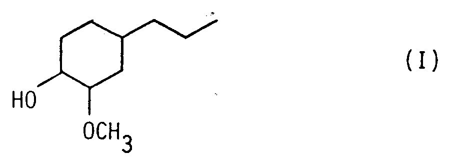
ou 2-méthoxy-4-propyl-1-cyclohexanol. Il s'agit d'un composé de structure connue mais pour lequel aucune mention n'a été faite dans l'art antérieur quant à ses propriétés olfactives éventuelles. Ce composé a en effet été décrit par S. W. Eachus et C. W. Dence [Holzforschung 29, 41-8 (1975)] en tant que produit obtenu par hydrogénation de l'isoeugénol lors d'une étude relative à l'hydrogénation de composes servant comme modèle de la lignine.

Nous avons constaté que le 2-méthoxy-4-propyl-1-cyclohexanol possédait des propriétés odorantes fort intéressantes et que, de ce fait, il pouvait être employé à titre d'ingrédient parfumant pour la préparation de parfums, bases parfumantes et produits parfumés.

Ses caractères odorants sont de type aromatique, vert et naturel. Son odeur rappelle le cerfeuil, le persil, le basilic et l'armoise. Doté d'une puissance moyenne, il offre l'avantage de pouvoir s'accorder avec une grande variété de coingrédients parfumants usuels sans déséquilibrer l'harmonie de la composition dans laquelle il est englobé. De par ses caractères odorants, le 2-méthoxy-4-propyl-1-cyclohexanol peut trouver un emploi tant en parfumerie fine qu'en parfumerie technique. C'est ainsi qu'il peut entrer dans des bases parfumantes concentrées pouvant servir à la préparation de parfums alcooliques, concentrés de parfums ou eaux de toilette, ou peut servir à parfumer des produits aussi variés que des savons de toilette, des cosmétiques, des crèmes pour les soins corporels, des shampoings. Il peut également trouver une utilisation dans le parfumage de poudres détergentes ou d'adoucisseurs textiles, de désodorisants d'air ambiant ou de matériaux variés, par exemple les plastiques.

Les proportions dans lesquelles le 2-méthoxy-4-propyl-1-cyclohexanol peut être employé pour produire les effets désirés varient dans une gamme de valeurs assez étendue. C'est ainsi que des concentrations de l'ordre de 0,5-1% sont parfaitement adaptées pour le parfumage de produits tels les savons ou les détergents. Ces concentrations peuvent être d'environ 4-10%, voire plus élevées, lorsque le produit est utilisé pour la préparation de bases parfumantes ou concentrés.

L'homme du métier sait par expérience que de telles valeurs de concentration n'ont qu'un caractère indicatif et que leur évaluation dépend de la nature des produits que l'on désire parfumer, de celle des coingrédients utilises dans une composition donnée et naturellement de l'effet odorant particulier que l'on souhaite obtenir. A titre de coingrédients, on peut utiliser des composés d'origine naturelle ou synthétique. Leur nature spécifique ainsi que leur énumération est ici superflue et référence est faite aux ouvrages spécialisés de l'art [voir par exemple : S. Arctander, Perfume and Flavor Chemicals, Montclair, N.J. (1969)].

Le 2-méthoxy-4-propyl-1-cyclohexanol peut être préparé comme indiqué plus haut, par hydrogénation de l'isoeugénol selon le procédé décrit par Eachus et Dence. Il peut également être obtenu par hydrogénation catalytique de l'eugénol à l'aide de catalyseurs métalliques tels le palladium ou le ruthénium sur support d'alumine, le ruthénium sur charbon actif ou le nickel de Raney. L'hydrogénation s'effectue sous pression et à cet effet une pression comprise entre environ 100 et 300 bar peut être employée. A ces valeurs de pression, on a employé des températures de l'ordre de 75-200°C, ce qui permet de réaliser des taux de conversion proche de 100% ainsi que d'excellents rendements.

La réaction peut s'effectuer dans un appareillage usuel au moyen d'un autoclave en acier inoxydable. Le 2-méthoxy-4-propyl-1-cyclohexanol ainsi obtenu peut être purifié, après séparation du mélange réactionnel, à l'aide des techniques courantes, par exemple par distillation fractionnée.

Le produit tel que représenté par la formule (I) sert à définir indifféremment chacun des isomères dont la structure peut être définie par l'une des formules suivantes : 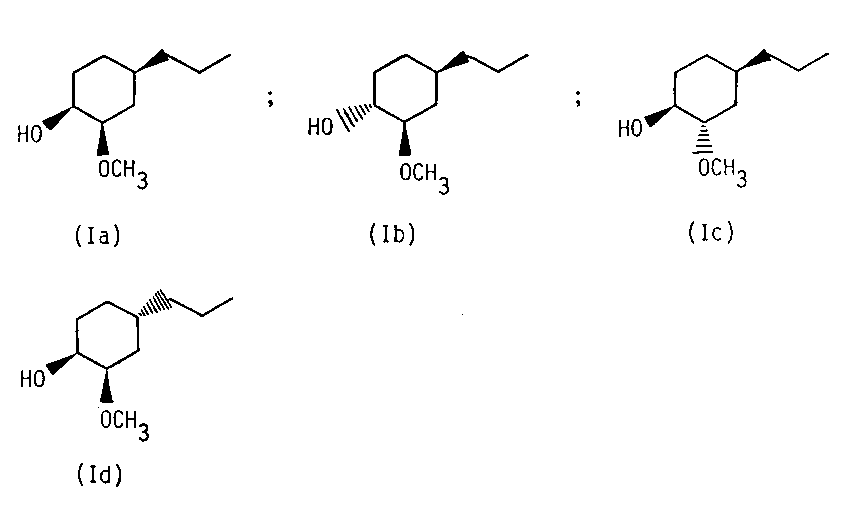
Ces différents isomères peuvent être séparés, par exemple par distillation fractionnée. Leurs caractères particuliers seront indiqués dans le détail dans les exemples spécifiques décrits ci-après.

Le rapport respectif de ces composés dans le mélange obtenu est fonction du système catalytique employé, toutefois la forme (Ia) est celle qui s'est révélée prépondérante dans tous les cas examinés.

Pour toute utilisation pratique en parfumerie, telle qu'envisagée dans la présente invention, le mélange constitué par ces différents isomères convient parfaitement. Si désiré, les différents composés peuvent être employés à l'état isolé après séparation. Quoique leurs qualités odorantes sont très apparentées, chaque isomère possède des caractéristiques propres. Il est apparu à l'usage que le composé (Ia) possédait les nuances les plus appréciées. Pour des raisons d'ordre pratique et économique, on préfère employer le mélange tel qu'obtenu par le procédé décrit plus haut ; on choisira toutefois des conditions de réaction pouvant fournir le produit dont le contenu en l'isomère (Ia) désiré est le plus favorable. A cet effet, l'utilisation du ruthénium sur alumine se révèle être le plus avantageux.

L'invention est illustrée de manière plus détaillée par les exemples suivants dans lesquels les températures sont indiquées en degrés centigrades et les abréviations ont le sens usuel dans l'art.

### Exemple 1

### Préparation de 2-méthoxy-4-propyl-1-cyclohexanol

1000 g d'eugénol et 10 g de ruthénium à 5% sur alumine dans 800 g d'éthanol ont été chargés dans un autoclave de 5 l. Après avoir purge l'appareil trois fois avec un courant d'azote (2 kg/cm²) et trois fois avec de l'hydrogène (2 kg/cm²), le mélange de réaction a été chauffé à 85° sous vigoureuse agitation. Le déroulement de la réaction a été suivi par des analyses chromatographiques successives et la réaction a lieu à 85° sous une pression d'hydrogène de 100 kg/cm². Une fois l'hydrogénation terminée, l'autoclave a été dépressurisé puis purgé à l'azote. Le produit a été ensuite filtré et le catalyseur récupéré pour un prochain emploi.
Après évaporation du solvant, on a récupéré 1010 g d'un produit brut qui, par distillation fractionnée sur une colonne garnie de 50 cm de longueur, a fourni à 49-55°/1,33 x 10² Pa, 910 g (rend. 86,8%) du produit désiré. Ce produit peut être ultérieurement séparé en ses composants isomériques à l'aide d'une distillation fractionnée en utilisant une colonne à bande tournante type Normag d'une longueur de 1 m et en appliquant un vide de 1,33 x 10² Pa.
Quatre fractions ont été ainsi recueillies.

| fraction | isomère | eb.[°C]/1,33x10² Pa |
|---|---|---|
| 1 | Ia | 43-45 |
| 2 | Id | 45-48 |
| 3 | Ib | 49-51 |
| 4 | Ic | 52-55 |

### Ia ou C-2-Méthoxy-C-4-propyl-R-1-cyclohexanol

- ¹H-RMN (360 MHZ) :: 4,1 (1H,m) ; 3,39 (3H,s) ; 3,15 (1H,m) ; 2,1 (1H,s) ; 1,97 (1H,m) ; 1,74 (1H,m) ; 1,4-1,2 (9H,m) ; 0,88 (3H,t) delta ppm ;
- ¹³C-RMN :: 81,20(d), 65,76(d), 55,75(q), 39,21(t), 35,83(d), 31,89(t), 29,96(t), 25,76(t), 20,00(t), 14,31(q) delta ppm ;
- SM :: 113, 129, 71, 41.

### Ib ou T-2-Méthoxy-T-4-propyl-R-1-cyclohexanol

- ¹H-RMN (360 MHZ) + D₂O :: 3,4 (3H,s) ; 3,37 (1H,m) ; 2,95 (1H,m) ; 2,12 (1H,m) ; 1,99 (1H,m) ; 1,72 (1H,m) ; 1,4-1,2 (6H,m) ; 0,95 (1H,m) ; 0,88 (3H,t) ; 0,80 (1H,m) delta ppm ;
- ¹³C-RMN :: 84,86(d), 73,99(d), 56,36(q), 38,91(t), 35,80(d), 35,03(t), 31,62(t), 30,59(t), 21,20(t), 14,29(q) delta ppm ;
- SM :: 113, 129, 71, 41.

### Ic ou T-2-Méthoxy-C-4-propyl-R-1-cyclohexanol

- ¹H-RMN (360 MHZ) :: 3,56 (1H,m) ; 3,35 (3H,s) ; 3,20 (1H,m) ; 2,31 (1H,s) ; 1,75 (3H,m) ; 1,6-1,2 (8H,m) ; 0,90 (3H,t) delta ppm ;
- ¹³C-RMN :: 80,52(d), 71,16(d), 56,21(q), 36,07(t), 31,84(t), 31,61(t), 25,56(t), 26,72(t), 20,38(t), 14,08(q) delta ppm ;
- SM :: 113, 129, 71, 41.

La pureté de la fraction Id (inférieur à 30%) ne nous a pas permis d'effectuer une analyse spectrale approfondie.

Les quatre isomères mentionnés ci-dessus ont également pu être mis en évidence par analyse gaz-chromatographique du mélange de réaction obtenu au moyen d'une colonne SPWAX 60 m ; progr. 80°(5')-220° ; 5°/min. Les résultats obtenus lors des différents essais d'hydrogénation sont résumés dans le tableau suivant :

**Tableau**

| | Catalyseur | P[bar] | T [°C] | Rend [%] | Rapport isomérique | | | |
|---|---|---|---|---|---|---|---|---|
| | | | | | (Ia) | (Ib) | (Ic) | (Id) |
| 1 | Pd*/Al₂O₃ | 280 | 200 | 99 | 44.3 | 18.4 | 12.2 | 25.1 |
| 2 | Ru*/Al₂O₃ | 100 | 85 | 87 | 67.6 | 22.1 | 2.6 | 7.7 |
| 3 | Ru*/C | 180 | 170 | 72 | 66.1 | 20.4 | 2.8 | 10.7 |
| 4 | Ni/Raney | 215 | 150 | 54 | 52.9 | 24.9 | 7.0 | 15.2 |

| | | | | | | | | |
|---|---|---|---|---|---|---|---|---|
| * 5% | | | | | | | | |

### Exemple 2

### Composition parfumante

Une composition parfumante de base a été préparée en mélangeant les ingrédients suivants (parties en poids) :

| | |
|---|---|
| Ald. hexylcinnamique | 260 |
| Acétate d'isononyle | 120 |
| Bergamote synthétique | 120 |
| Acétate de p-tert-butylcyclohexyle | 100 |
| Lilial (marque enregistrée) ¹⁾ | 80 |
| Tétrahydrolinalol | 60 |
| alpha-Terpinéol | 40 |
| Salicylate de benzyle | 40 |
| Alcool hydrotropique | 40 |
| Diméthyl-cyclohexène-carbaldéhyde à 10% * | 30 |
| Essence de menthe citrata | 20 |
| Essence de lavande | 20 |
| Prasinate ²⁾ | 10 |
| Verdanthiol ³⁾ à 50% * | 10 |
| Ald. méthyl-nonylique à 10% * | 10 |
| Total | 9̅6̅0̅ |

| | |
|---|---|
| 1) L. Givaudan ; ald. p-tert-butyl-alpha-méthyl hydrocinnamique | |
| 2) Firmenich SA ; 2-acétyl-4-méthyl-4-penténoate d'éthyle | |
| 3) L. Givaudan ; Lilial-méthylanthranilate | |
| * dans le phtalate d'éthyle | |

En ajoutant à 96 g de la composition de base ainsi obtenue 4 g de 2-méthoxy-4-propyl-1-cyclohexanol, obtenu conformément à l'Exemple 1, on obtient une nouvelle composition dont le caractère vert, aromatique est beaucoup plus prononcé et élégant. La nouvelle composition possède en outre un pouvoir de diffusion amélioré et son caractère est plus arrondi.

### Exemple 3

### Composition parfumante

Une composition parfumante de base a été préparée en mélangeant les ingrédients suivants (parties en poids) :

| | |
|---|---|
| Estragol | 350 |
| Linalol | 200 |
| Géraniol | 120 |
| Basilex ¹⁾ à 10% * | 100 |
| Limonène | 60 |
| delta³-Carène | 40 |
| Eugénol | 20 |
| Méthyleugénol | 10 |
| Isoeugénol | 10 |
| Ald. anisique | 10 |
| alpha-Pinène | 10 |
| Essence de fenouil douce | 10 |
| Total | 9̅4̅0̅ |

| | |
|---|---|
| * dans le phtalate d'éthyle | |
| 1) Firmenich SA ; 3-méthyl-9-méthylène-tricyclo[5.2.1.0^{2,6}]déc-3-ène-8-exo-yle acétate | |

En ajoutant à 94 g de la composition de base ainsi obtenue 6 g de 2-méthoxy-4-propyl-1-cyclohexanol, le caractère aromatique de la base acquiert une note "basilic" tout en devenant plus naturelle, fraîche et douce.

## Revendications

1. Utilisation du 2-méthoxy-4-propyl-1-cyclohexanol à titre d'ingrédient parfumant.

2. Composition parfumante caractérisée en ce qu'elle contient à titre d'ingrédient parfumant le 2-méthoxy-4-propyl-1-cyclohexanol.

3. Produit parfumé caractérisé en ce qu'il contient à titre d'ingrédient parfumant le 2-méthoxy-4-propyl-1-cyclohexanol.

4. A titre de produit parfumé selon la revendication 3, un détergent, un adoucissant textile, un savon ou un désodorisant d'air ambiant

5. A titre de produit parfumé selon la revendication 3, un cosmétique, un shampoing ou un désodorisant corporel.

## Claims

1. Use of 2-methoxy-4-propyl-1-cyclohexanol as perfuming ingredient.

2. Perfuming composition characterized in that it contains as perfuming ingredient 2-methoxy-4-propyl-1-cyclohexanol.

3. Perfumed article, characterized in that it contains as perfuming ingredient 2-methoxy-4-propyl-1-cyclohexanol.

4. As a perfumed article according to claim 3, a detergent, a fabric softener, a soap or an air deodorant.

5. As a perfumed article according to claim 3, a cosmetic preparation, a shampoo or a body deodorant.

## Patentansprüche

1. Verwendung von 2-Methoxy-4-propyl-1-cyclohexanol als Riechstoffbestandteil.

2. Riechstoffzusammensetzung, dadurch gekennzeichnet, dass sie als Riechstoffbestandteil 2-Methoxy-4-propyl-1-cyclohexanol enthält.

3. Parfümiertes Produkt, dadurch gekennzeichnet, dass es als Riechstoffbestandteil 2-Methoxy-4-propyl-1-cyclohexanol enthält.

4. Ein Reinigungsmittel, ein Textilauffrischungsmittel, eine Seife oder ein Raumluftverbesserer als parfümiertes Produkt gemäss Patentanspruch 3.

5. Ein Kosmetikum, ein Shampoo oder ein Körperdesodorant als parfümiertes Produkt gemäss Patentanspruch 3.
